# DEMANDE DE BREVET EUROPEEN

(11) **EP 0 639 552 A1**
(43) Date de publication de la demande: **22.02.1995**
(21) Numéro de dépôt: 94401866.2
(22) Date de dépôt: 18.08.1994
(51) Int. Cl.: C07C 35/36, C07C 35/37, C07C 69/013, C07D 303/16, C07D 307/77

(54) **Nouveaux dérivés du naphthalène, leur procédé de préparation et les compositionspharmaceutiques qui les contiennent**

(30) Priorité: 18.08.1993 FR 9310069
(71) Demandeur: ADIR ET COMPAGNIE, F-92415 Courbevoie Cédex (FR)
(72) Inventeur: Billington, David, F-92300 Levallois Perret (FR); Perron-Sierra, Françoise, F-75015 Paris (FR); Picard, Isabelle, F-75015 Paris (FR); Duhault, Jacques, F-78290 Croissy sur seine (FR); Espinal, Joseph, 92300 Levallois-Perret (FR)

(57) **Abrégé**

Composés de formule (I) : 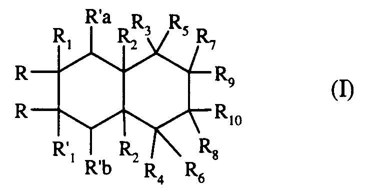
dans laquelle :
- R₁, R'₁ et R₂ sont choisis indépendamment l'un de l'autre parmi l'hydrogène et un radical alkyle, les deux groupements R₂ étant en position "cis" par rapport aux cycles,
- et R, R₃, R₄, R₅, R₆, R₇, R₈, R₉, R₁₀, R'ₐ et R'_{b} sont tels que définis dans la description.

Ces composés trouvent leur application en thérapeutique dans le traitement du diabète de l'insulinorésistance, des maladies cardiovasculaires et des insulinomes.

## Description

La présente invention concerne de nouveaux dérivés du naphtalène partiellement ou totalement hydrogénés, substitués par au moins deux groupements hydroxy, alkoxy ou acyloxy, leur procédé de préparation et les compositions pharmaceutiques qui les contiennent.

La demanderesse a présentement découvert que ces nouveaux dérivés du naphtalène possèdent de remarquables propriétés pharmacologiques. Ils se révèlent notamment être de puissants régulateurs de sécrétion d'insuline et à ce titre peuvent être utilisés dans le traitement du diabète, de l'insulinorésistance, des maladies cardio-vasculaires et des insulinomes.

Plus particulièrement, la présente invention concerne les composés de formule (I): 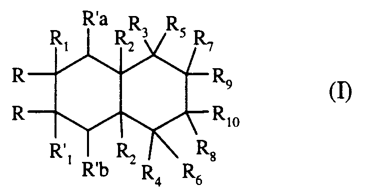
dans laquelle:
- R représente un radical choisi parmi hydroxy, alkoxy et acyloxy,
- R₁, R'₁ et R₂ sont choisis indépendamment l'un de l'autre parmi l'hydrogène et un radical alkyle, les deux groupements R₂ étant en position "cis" par rapport aux cycles,
- R₃ et R₄ représentent l'hydrogène ou forment ensemble un pont méthylène ou éthylène,
- R₅ et R₆, indépendamment l'un de l'autre sont choisis parmi l'hydrogène, le radical hydroxy, un radical alkoxy, un radical acyloxy et un radical alkyle,
- R₇ et R₈, indépendamment l'un de l'autre, sont choisis parmi l'hydrogène et un radical alkyle,
- R₉, R₁₀, R'ₐ et R'_{b}
   - sont choisis, indépendamment les uns des autres, parmi l'hydrogène, le radical hydroxy, un radical alkoxy et un radical acyloxy,
   - ou bien R₉ et R₁₀ forment ensemble une double liaison ou un cycle avec un atome d'oxygène et R'ₐ et R'_{b} sont tels que définis précédemment,
   - ou bien R₉ et R'ₐ forment ensemble un cycle avec un atome d'oxygène et R₁₀ et R'_{b} sont tels que définis précédemment,
   - ou bien R₁₀ et R'_{b} forment ensemble un cycle avec un atome d'oxygène et R₉ et R'ₐ sont tels que définis précédemment,

étant entendu que les termes "alkyle", "alkoxy" et "acyloxy" désignent des groupements linéaires ou ramifiés, comportant de 1 à 6 atomes de carbone,
leurs stéréoisomères, ainsi que leurs éventuels sels d'addition à une base, pharmaceutiquement acceptables.

La présente invention s'étend également au procédé de préparation des composés de formule (I), caractérisé en ce que l'on engage dans une réaction de Diels-Alder, dans un solvant apolaire tel que le toluène, sous atmosphère inerte, à chaud, par exemple à des températures de 40 à 80°C, une quinone de formule (II) et un diène de formule (III): 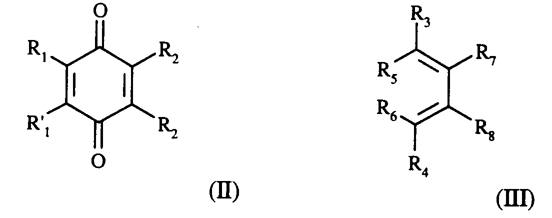
dans lesquelles R₁, R'₁, R₂, R₃, R₄, R₅, R₆, R₇ et R₈ sont tels que définis précédemment,
de manière à obtenir l'intermédiaire de formule (IV) : 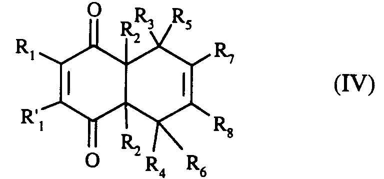
dans laquelle R₁, R'₁, R₂, R₃, R₄, R₅, R₆, R₇ et R₈ sont tels que définis précédemment,
qui, après séparation des éventuels stéréoisomères selon une technique classique de séparation, est ensuite soumis à un agent de réduction, par exemple l'hydrure double de lithium et d'aluminium ou le borohydrure de sodium, en présence de chlorure de cérium, en solvant anhydre, tel que le méthanol, le dichlorométhane, le diméthylformamide ou le tétrahydrofurane, à température appropriée, soit de 0 à 22°C, afin d'obtenir le diol de formule (Vₐ): 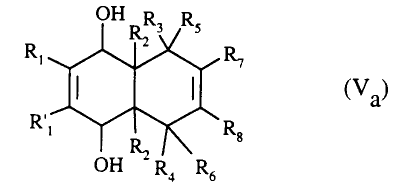
dans laquelle R₁, R'₁, R₂, R₃, R₄, R₅, R₆, R₇ et R₈ sont tels que définis précédemment,
qui, après séparation des éventuels stéréoisomères selon une technique classique de séparation, peut être soumis à l'action d'un agent d'oxydation tel que l'acide chloro-3-perbenzoïque, pour conduire aux composés de formules (Vₐ₁) et (Vₐ₂), séparés selon une technique classique de séparation: 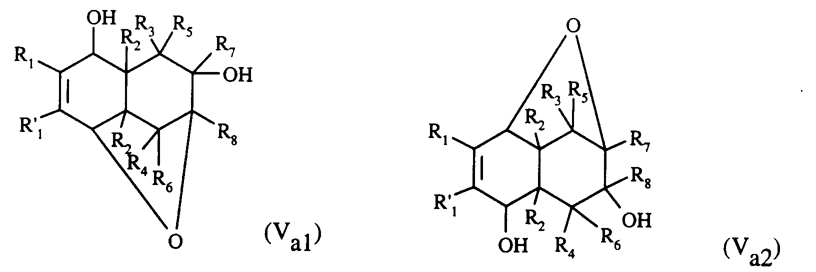
dans lesquelles R₁, R'₁, R₂, R₃, R₄, R₅, R₆, R₇ et R₈ sont tels que définis précédemment,
composés de formules (Vₐ), (Vₐ₁) et (Vₐ₂) dont les fonctions hydroxy peuvent être alkylées ou acylées selon des techniques classiques d'alkylation ou d'acylation respectivement,
les composés de formules (Vₐ), (Vₐ₁) et (Vₐ₂) ainsi que leurs éventuels produits d'alkylation et d'acylation étant ensuite hydroxylés,
- dans le cas d'une cis-hydroxylation par des méthodes classiques, telles que l'osmylation (tétroxyde d'osmium en présence d'un co-oxydant), ou l'oxydation par le permanganate de potassium, dans les solvants polaires, tels que le tertiobutanol, le tétrahydrofurane, l'acétone, l'eau ou la pyridine,
- dans le cas d'une trans-hydroxylation par une réaction d'époxydation, par exemple à l'aide d'acide chloro-3-perbenzoïque, suivie d'un traitement en milieu acide, par exemple acide trifluoroacétique ou acide acétique, pour conduire respectivement, après séparation des éventuels stéréoisomères selon des techniques classiques de séparation, aux composés de formules (VIₐ), (VIₐ₁), et (VIₐ₂) :

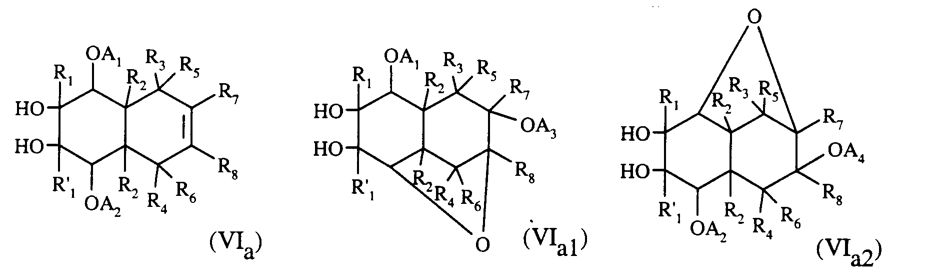

dans laquelle R₁, R'₁, R₂, R₃, R₄, R₅, R₆, R₇ et R₈ sont tels que définis précédemment, et A₁, A₂, A₃ et A₄ représentent indépendamment les uns des autres l'hydrogène ou un groupement alkyle ou acyle tels que définis précédemment,
dont les fonctions hydroxy peuvent être alkylées ou acylées selon des méthodes classiques, l'ensemble des composés de formules (VIₐ), (VIₐ₁) et (VIₐ₂) ainsi que leurs produits d'alkylation et d'acylation formant respectivement l'ensemble des composés de formules (VI), (VI₁) et (VI₂) : 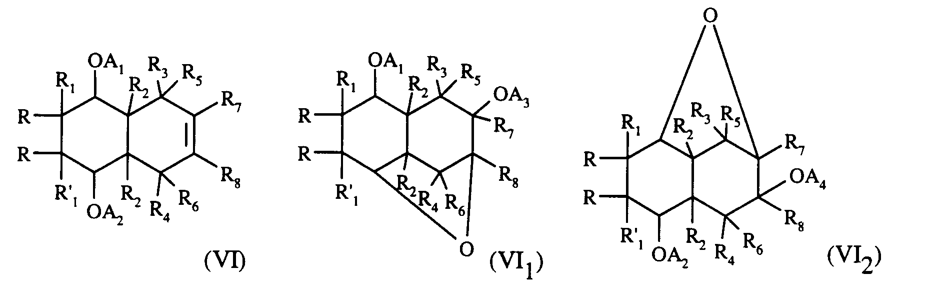
dans lesquelles R, R₁, R'₁, R₂, R₃, R₄, R₅, R₆, R₇, R₈, A₁, A₂, A₃ et A₄ sont tels que définis précédemment,
les composés de formule (VI) pouvant être:
- soit hydrogénés, par exemple sous pression atmosphérique, en présence de catalyseur tel que le palladium sur charbon ou le platine sur charbon, en composés de formule (VI_{b}) : 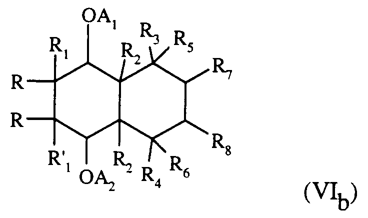 dans laquelle R, R₁, R'₁, R₂, R₃, R₄, R₅, R₆, R₇, R₈, A₁ et A₂ sont tels que définis précédemment,
- soit hydroxylés, par exemple par action de tétroxyde d'osmium ou de permanganate de potassium, dans le cas d'une cis-hydroxylation, ou par action de pentoxyde de vanadium, dans le cas d'une trans-hydroxylation, en composés de formule (VI_{C1}): 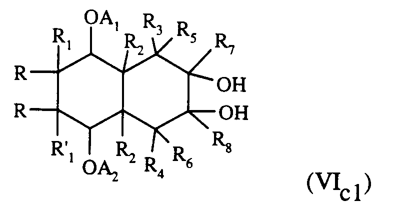 dans laquelle R, R₁, R'₁, R₂, R₃, R₄, R₅, R₆, R₇, R₈, A₁ et A₂ sont tels que définis précédemment,
   dont les fonctions hydroxy peuvent être alkylées ou acylées selon des techniques classiques, les composés de formule (VI_{C1}) ainsi que leurs produits d'acylation et d'alkylation formant l'ensemble des composés de formule (VI_{c}) : 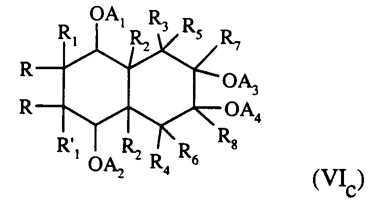 dans laquelle R, R₁, R'₁, R₂, R₃, R₄, R₅, R₆, R₇, R₈, A₁, A₂, A₃ et A₄ sont tels que définis précédemment,
- soit mis en présence d'un agent oxydant tel que l'acide chloro-3-perbenzoïque, afin d'obtenir le composé de formule (VI_{d}): 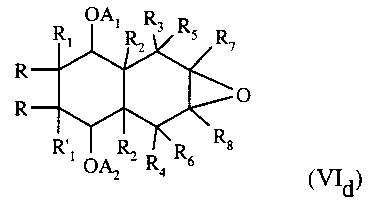 dans laquelle R, R₁, R'₁, R₂, R₃, R₄, R₅, R₆, R₇, R₈, A₁ et A₂ sont tels que définis précédemment,

l'ensemble des composés de formules (VI), (VI₁), (VI₂), (VI_{b}), (VI_{c}) et (VI_{d}) formant l'ensemble des composés de formule (I) qui sont, si on le souhaite, purifiés selon une technique classique de purification, et séparés en leurs stéréoisomères éventuels selon des techniques classiques de séparation et transformés en leurs éventuels sels d'addition à une base, pharmaceutiquement acceptables.

La demanderesse a découvert que les composés de l'invention se montrent très utiles pour toutes les pathologies présentant une anomalie de la sécrétion d'insuline, soit un déficit, tel que dans le diabète ou l'insulinorésistance, soit une hypersécrétion d'insuline, facteur de risque rencontré dans les maladies cardiovasculaires, facteur également présent dans le cas d'insulinomes.

La présente invention a également pour objet les compositions pharmaceutiques contenant au moins un composé de formule (I), ou un de ses sels d'addition à une base, pharmaceutiquement acceptables, seul ou en combinaison avec un ou plusieurs excipients inertes et non toxiques. Parmi les compositions pharmaceutiques selon l'invention, on pourra citer plus particulièrement celles qui conviennent à l'administration orale, parentérale, nasale, rectale, perlinguale, oculaire ou pulmonaire et notamment les préparations injectables, les aérosols, les gouttes oculaires ou nasales, les comprimés simples, pelliculés ou dragéifiées, les gélules, les capsules, les suppositoires, les crèmes, pommades, gels dermiques...

La posologie utile varie selon l'âge et le poids du patient, la voie d'administration, la nature de l'affection et des traitements éventuels associés et s'échelonne entre 0,5 mg et 1 g par 24 heures.

Les exemples suivants illustrent l'invention sans toutefois la limiter en aucune façon.

Les matières premières sont disponibles ou préparées à partir de modes opératoires connus.

La numérotation adoptée pour la nomenclature des dérivés naphtaléniques des exemples suivants est conforme à la numérotation de l'IUPAC. à savoir:

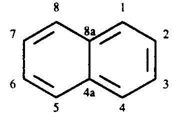

### EXEMPLE 1 : 1,4,4aβ,5,6,7,8,8aβ-Octahydro-5α,6β,7β,8α-tétraacétoxy-1β,4β-éthanonaphtalène

- **Etape α** : 1,4,4aβ,5,8,8aβ-Hexahydro-5,8-dioxo-1β,4β-éthanonaphtalène
   22 ml (230,9 mmoles) de 1,3-cyclohexadiène sont additionnés goutte à goutte à une solution de 25 g (230,9 mmoles) de para-benzoquinone dans 200 ml de toluène, sous atmosphère inerte et à température ambiante. Le milieu réactionnel est alors agité à 40°C pendant 16 heures. La réaction est suivie par chromatographie sur couche mince. En fin de réaction, le milieu réactionnel est concentré sous pression réduite. Le résidu, trituré dans l'éther de pétrole, cristallise pour donner 39,4 g d'une poudre verte.
   Point de fusion: 93-95°C
   Rendement : 91 %
- **Etape β** : 1,4,4aβ,5,8,8aβ-Hexahydro-5α,8α-dihydroxy-1β,4β-éthanonaphtalène
   10 g (53,1 mmoles) du composé obtenu à l'étape α sont dissous dans 2 litres d'un mélange équivolumique de dichlorométhane et de méthanol. 19,88 g (53,4 mmoles) de chlorure de cérium (III) heptahydraté sont additionnés en une seule fois à température ambiante. L'ensemble est alors agité jusqu'à dissolution totale. 2 g (53,1 mmoles) de borohydrure de sodium sont alors ajoutés, par petites portions, à température ambiante.
   Lorsque la réaction, suivie par chromatographie sur couche mince, est terminée, le milieu réactionnel est concentré sous pression réduite. L'huile jaunâtre obtenue est alors reprise dans l'éther et 7,2 g du composé attendu cristallisent sous forme d'une poudre blanche.
   Point de fusion : 130-132°C
   Rendement : 70 %
- **Etape γ** : 1,4,4aβ,5,6,7,8,8aβ-Octahydro-5α,6β,7β,8α-tétra-acétoxy-1β,4β-éthanonaphtalène
   11 g (57,2 mmoles) du composé obtenu à l'étape β sont dissous dans 770 ml d'un mélange tertiobutanol/eau/pyridine (25:7:1). 35,5 ml d'une solution à 2,5 % en poids dans le tertiobutanol (2,87 mmoles) de tétroxyde d'osmium puis 9,6 g (86,1 mmoles) de N-oxyde de triméthylamine sont ajoutés. L'ensemble est chauffé à 60°C et maintenu sous agitation magnétique pendant 20 heures. A la fin de la réaction, l'excès d'agent oxydant est détruit par addition de 40 ml d'une solution aqueuse à 20 % d'hydrogénosulfonate de sodium. Le milieu réactionnel est alors concentré sous pression réduite. L'huile brune obtenue est alors dissoute dans 100 ml de pyridine. 100 ml d'anhydride acétique sont alors ajoutés goutte à goutte et l'ensemble est maintenu sous agitation pendant 12 heures. Le milieu réactionnel est concentré sous pression réduite puis chromatographié sur colonne de gel de silice (éluant : hexane/acétate d'éthyle, 2:1). 14 g du composé attendu accompagné de son isomère trans dans une proportion de 90:10 sont obtenus.
   Rendement global : 62 %

### EXEMPLE 2 : 1,2,3,4,4aβ,5,6,7,8,8aβ-Décahydro-2β,3β-dihydroxy-5α,6β,7β,8α-tétraacétoxy-1β,4β-éthanonaphtalène

10 g (25,4 mmoles) du composé obtenu à l'exemple 1 sont dissous dans 335 ml d'un mélange tertiobutanol/eau/pyridine (25:7:1). 16,3 ml (1,3 mmoles) d'une solution à 2,5 % en poids dans le tertiobutanol de tétroxyde d'osmium sont ensuite ajoutés, puis 4,23 g (38,1 mmoles) de N-oxyde de triméthylamine. Le mélange réactionnel est chauffé à 60°C et maintenu sous agitation magnétique jusqu'à oxydation maximale. L'excès d'agent oxydant est détruit par addition de 20 ml d'une solution aqueuse à 20 % d'hydrogénosulfate de sodium. L'ensemble est alors concentré sous pression réduite. L'huile brune obtenue est reprise dans le dichlorométhane et lavée avec une solution saturée en chlorure de sodium. Le traitement habituel des phases organiques fournit, après purification sur colonne de gel de silice (éluant: hexane/acétate d'éthyle ; 1:4), 5,5 g du produit attendu sous forme de poudre blanche.
Point de fusion : 98-100°C
Rendement : 83 %

### EXEMPLE 3 : 1,2,3,4,4aβ,5,6,7,8,8aβ-Décahydro-2β,3β,5α,6β,7β,8α-hexahydroxy-1β,4β-éthanonaphtalène

2 g (4,7 mmoles) du composé obtenu dans l'exemple 2 sont dissous dans 100 ml de méthanol et soumis à une désacétylation en présence de résine amberlite (OH⁻).La réaction est suivie par chromatographie sur couche mince. Après filtration, la solution est concentrée sous pression réduite pour donner 828 mg d'une poudre blanche correspondant au produit attendu.
Point de fusion: 188-192°C
Rendement: 69 %

### EXEMPLE 4 : 1,4,4aβ,5,6,7,8,8aβ-Octahydro-5α,6β,7β,8α-tétrahydroxy-1β,4β-éthanonaphtalène

Ce composé est obtenu de manière identique à celle décrite dans l'exemple 1, la synthèse étant stoppée dans l'étape γ avant l'acétylation du composé.
Point de fusion: 172°C (décomposition)

### EXEMPLE 5 : 1,2,3,4,4aβ,5,6,7,8,8aβ-Décahydro-3α,5α-époxyméthano-2β,6β,7β,8α-tétraacétoxy-1β,4β-éthanonaphtalène

- **Etape α** : 1,4,4aβ,5,6,7,8,8aβ-Octahydro-3α,5α-époxyméthano-2β,8α-dihydroxy-1β,4β-éthanonaphtalène
   13,4 g (69,7 mmoles) du composé obtenu dans l'exemple 1, étape β, sont dissous dans 1 litre de dichlorométhane. A cette solution refroidie par un bain de glace, 18,9 g (76,7 mmoles) d'acide chloro-3-perbenzoïque sont ajoutés par petites portions. L'ensemble est maintenu 1 heure sous agitation à 0°C, puis à température ambiante jusqu'à disparition totale du produit de départ (suivi de la réaction par chromatographie sur couche mince). L'ensemble est alors refroidi à -78°C et le précipité formé est filtré rapidemment. Le filtrat, concentré sous pression réduite, fournit une huile jaunâtre qui est chromatographiée sur colonne de gel de silice. (éluant: hexane/acétate d'éthyle 1:4) 10,3 g de composé attendu sont alors obtenus sous forme de poudre blanche.
   Point de fusion : 140-143°C
   Rendement : 71 %
- **Etape β** : 1,2,3,4,4aβ,5,6,7,8,8aβ-Décahydro-3α,5α-époxyméthano-2β,6β, 7β,8α-tétraacétoxy-1β,4β-éthanonaphtalène
   6 g (28 mmoles) du composé obtenu à l'étape précédente sont soumis à l'action de 2,93 ml (0,29 mmoles) d'une solution à 2,5 % en masse dans le tertiobutanol de tétroxyde d'osmium, en présence de 5 g (43,2 mmoles) de N-oxyde de N-méthylmorpholine, dans 80 ml d'un mélange tétrahydrofurane/tertiobutanol/eau (20:13:6). Le produit brut de la réaction est acétylé selon la méthode décrite dans l'exemple 1, étape γ.

Le milieu réactionnel est concentré sous pression réduite pour être ensuite chromatographié sur colonne de gel de silice (éluant : hexane/acétate d'éthyle ; 4:1) pour donner le produit attendu.
Point de fusion : 154-157°C

### EXEMPLE 6 : 1,2,3,4,4aβ,5,6,7,8,8aβ-Décahydro-3α,5α-époxyméthano-2β,6β,7β,8α-tétrahydroxy-1β,4β-éthanonaphtalène

Procédé identique à celui décrit pour l'exemple 3 à partir du composé obtenu à l'exemple 5.
Point de fusion : 208-210 °C

Les composés des exemples 7 à 11 ont été obtenus en opérant comme décrit dans l'exemple 1, en remplaçant le cyclohexadiène par le diène approprié.

### EXEMPLE 7 : 1,4,4aβ,5,6,7,8,8aβ-Octahydro-5α,6β,7β,8α-tétra-acétoxy-1β,4β-méthanonaphtalène

### EXEMPLE 8 : 1,4,4aβ,5,6,7,8,8aβ-Octahydro-1α,4α,5α,6β,7β,8α-hexa-acétoxynaphtalène

### EXEMPLE 9 : 1,4,4aβ,5,6,7,8,8aβ-Octahydro-5α,6β,7β,8α-tétra-acétoxy-2,3-diméthylnaphtalène

### EXEMPLE 10 : 1,4,4aβ,5,6,7,8,8aβ-Octahydro-5α,6β,7β,8α-tétra-acétoxy-1α-méthyl-4α-isopropylnaphtalène

### EXEMPLE 11 : 1,4,4aβ,5,6,7,8,8aβ-Octahydro-5α,6β,7β,8α-tétra-acétoxy-1α-méthyl-4α-isopropyl-1β,4β-éthanonaphtalène

En procédant selon le mode opératoire décrit dans l'exemple 4, les composés des exemples 12 à 16 sont obtenus, à partir des composés des exemples 7 à 11, respectivement.

### EXEMPLE 12 : 1,4,4aβ,5,6,7,8,8aβ-Octahydro-5α,6β,7β,8α-tétrahydroxy-1β,4β-méthanonaphtalène

### EXEMPLE 13 : 1,4,4aβ,5,6,7,8,8aβ-Octahydro-1α,4α,5α,6β,7β,8α-hexahydroxynaphtalène Point de fusion : 59-62°C

### EXEMPLE 14 : 1,4,4aβ,5,6,7,8,8aβ-Octahydro-5α,6β,7β,8α-tétrahydroxy-2,3-diméthylnaphtalène

### EXEMPLE 15 : 1,4,4aβ,5,6,7,8,8aβ-Octahydro-5α,6β,7β,8α-tétrahydroxy-1α-méthyl-4α-isopropylnaphtalène

### EXEMPLE 16 : 1,4,4aβ,5,6,7,8,8aβ-Octahydro-5α,6β,7β,8α-tétrahydroxy-1α-méthyl-4α-isopropyl-1β,4β-éthanonaphtalène Point de fusion : 183-184 °C

Les exemples 17 à 22 sont obtenus selon le mode opératoire décrit dans l'exemple 3, à partir de para-benzoquinone et du diène approprié.

### EXEMPLE 17 : 1,2,3,4,4aβ,5,6,7,8,8aβ-Décahydro-2β,3β,5α,6β,7β,8α-hexahydroxy-1β,4β-méthanonaphtalène Point de fusion : 162-164 °C

### EXEMPLE 18 : 1,2,3,4aβ,5,6,7,8,8aβ-Décahydro-1α,2β,3β,4α,5α,6β,7β,8α-octahydroxynaphtalène Point de fusion : 262 °C (décomposition)

### EXEMPLE 19 : 1,2,3,4,4aβ,5,6,7,8,8aβ-Décahydro-2β,3β,5α,6β,7β,8α-hexahydroxy-2α,3α-diméthylnaphtalène Point de fusion : 212-214 °C

### EXEMPLE 20 : 1,2,3,4,4aβ,5,6,7,8,8aβ-Décahydro-2β,3α,5α,6β,7β,8α-hexahydroxy-2α,3β-diméthylnaphtalène Point de fusion : 215-217 °C

### EXEMPLE 21 : 1,2,3,4,4aβ,5,6,7,8,8aβ-Décahydro-2β,3β,5α,6β,7β,8α-hexahydroxy-1α-méthyl-4α-isopropylnaphtalène

### EXEMPLE 22 : 1,2,3,4,4aβ,5,6,7,8,8aβ-Décahydro-2β,3β,5α,6β,7β,8α-hexahydroxy-1α-méthyl-4α-isopropyl-1β,4β-éthanonaphtalène Point de fusion: (produit lyophilisé)

¹H RMN (DMSO), δ(ppm) : 5,60 (2H, m) ; 4,8 (1H, d) ; 4,75 (1H, d) ; 4,48 (1H, d) ; 4,30 (2H, m) ; 4,1 (1H, t) ; 3,85 (2H, m) ; 3,75 (1H, m) ; 3,68 (1H, m) ; 2,05 (1H, dd); 1,9 (1H, dd) ; 1,8 à 1,5 (3H, m) ; 1,15 (1H, m) ; 0,9 (3H, d,) ; 0,85 (3H, s) ; 0,82 (1H, m).

### EXEMPLE 23 : 1,2,3,4,4aβ,5,6,7,8,8aβ-Décahydro-5α,6β,7β,8α-tétra-acétoxy-1β,4β-éthanonaphtalène

800 mg de composé obtenu à l'exemple 1 sont dissous dans 30 ml d'un mélange méthanol/acétate d'éthyle (1:1). 80 mg de palladium sur charbon sont alors ajoutés. Le mélange est agité en présence d'hydrogène à pression atmosphérique jusqu'à disparition totale du produit de départ. Après filtration sur célite, le filtrat est concentré sous pression réduite. 700 mg du composé attendu sont obtenus.
Rendement: 90 %

¹H RMN (DMSO), δ(ppm) : 5,35 (2H, m) ; 5,24 (2H, m) ; 2,45 (2H, m) ; 2,00 (12H, s) ; 1,7 à 1,45 (10H, m).

En procédant de la même manière, à partir des composés obtenus aux exemples 8 et 11, les exemples 24 et 25, respectivement, sont obtenus.

### EXEMPLE 24 : 1,2,3,4,4aβ,5,6,7,8,8aβ-Décahydro-1α,4α,5α,6β,7β,8α-hexa-acétoxynaphtalène

### EXEMPLE 25 : 1,2,3,4,4aβ,5,6,7,8,8aβ-Décahydro-5α,6β,7β,8α-tétra-acétoxy-1α-méthyl-4α-isopropyl-1β,4β-éthanonaphtalène

Les 3 exemples suivants sont obtenus par désacétylation des composés obtenus aux exemples 23, 24 et 25 respectivement, selon le mode opératoire décrit à l'exemple 4.

### EXEMPLE 26 : 1,4,4aβ,5,6,7,8,8aβ-Décahydro-5α,6β,7β,8α-tétrahydroxy-1β,4β-éthanonaphtalène Point de fusion : 182-185 °C (Décomposition)

### EXEMPLE 27 : 1,4,4aβ,5,6,7,8,8aβ-Décahydro-1α,4α,5α,6β,7β,8α-hexahydroxynaphtalène Point de fusion: (mousse) (55°C)

### EXEMPLE 28 : 1,2,3,4,4aβ,5,6,7,8,8aβ-Décahydro-5α,6β,7β,8α-tétrahydroxy-1α-méthyl-4α-isopropyl-1β,4β-éthanonaphtalène

### EXEMPLE 29 : 1,2,3,4,4aβ,5,6,7,8,8aβ-Décahydro-2β,3β-époxy-5α,6β,7β,8α-tétra-acétoxy-1β,4β-éthanonaphtalène

1 g (2,6 mmoles) de composé obtenu à l'exemple 1 sont dissous dans 40 ml de dichlorométhane. A cette solution, refroidie par un bain de glace, sont ajoutés 1,42 g (5,76 mmoles) d'acide chloro-3-perbenzoïque à 70 % par petites portions. La solution est agitée à 0°C pendant 1 heure, puis à température ambiante jusqu'à disparition totale du produit de départ (réaction suivie par chromatographie sur couche mince). Le milieu réactionnel est ensuite refroidi à -78°C, et le précipité formé est filtré rapidement. Le filtrat est alors concentré sous pression réduite et l'huile jaunâtre ainsi obtenue est chromatographiée sur colonne de gel de silice (éluant : dichlorométhane/acétate d'éthyle 10: 1). 140 mg du composé attendu sont obtenus sous forme de poudre blanche.
Rendement : 90 %
Point de fusion : 186-188 °C

### EXEMPLE 30 : 1,2,3,4,4aβ,5,6,7,8,8aβ-Décahydro-2β,3β-dihydroxy-5α,6β,7β,8α-tétra-acétoxy-2α,3α-diméthylnaphtalène

Le composé obtenu à l'exemple 9 est engagé dans la réaction d'hydroxylation décrite dans l'exemple 2.
Point de fusion : 169-171 °C

En procédant comme décrit pour la préparation de l'exemple 6, et en isolant à chaque fois l'isomère concerné, les composés suivants sont obtenus.

### EXEMPLE 31 : 1,2,3,4,4aβ,5,6,7,8,8aβ-Décahydro-2β,6β,7α,8α-tétrahydroxy-3α,5α-époxyméthano-1β,4β-éthanonaphtalène Point de fusion : 208-211 °C

### EXEMPLE 32 : 1,2,3,4,4aβ,5,6,7,8,8aβ-Décahydro-2β,6β,7β,8α-tétrahydroxy-3α,5α-époxyméthano-1α-méthyl-4α-isopropyl- 1β,4β-éthanonaphtalène Point de fusion : (Lyophilisat)

¹H RMN (DMSO), δ(ppm) : 4,78 (1H, d) ; 4,5 (1H, d) ; 4,3 (1H, d) ; 4,15 (1H,d) ; 3,88 (1H, m) ; 3,8 (2H, m) ; 3,48 (2H, m) ; 3,3 (1H, m) ; 2,26 (1H, m); 2,05 (1H, m) ; 1,68 (1H, m) 1,65 à 1,45 (3H, m); 1,03 (3H, s) ; 0,85 (3H, d) ; 0,82 (1H, m).

### EXEMPLE 33 : 1,2,3,4,4aβ,5,6,7,8,8aβ-Décahydro-2β,6β,7β,8β-tétrahydroxy-3α,5α-époxyméthano-1α-isopropyl-4α-méthyl-1β,4β-éthanonaphtalène Point de fusion: (Lyophilisat)

¹H RMN (DMSO), δ(ppm) : 5,25 (1H, d) ; 5,05 (1H, d) ; 4,75 (1H, d) ; 4,65 (1H, d) ; 4,05 (1H, m) ; 4,0 (1H, m) ; 3,7 (1H, m) ;3,45 (2H, m) ; 3,22 (1H, m); 2,15 (1H, m); 2,0 (1H, m) ; 1,7 (1H, m) ; 1,6 à 1,5 (4H, m) ; 0,98 (3H, d) ; 0,95 (3H, s) ; 0,88 (3H, d).

### EXEMPLE 34 : 1,2,3,4,4aβ,5,6,7,8,8aβ-Décahydro-2β,6β,7α,8β-tétrahydroxy-3α,5α-époxyméthano-1α-isopropyl-4α-méthyl-1β,4β-éthanonaphtalène Point de fusion: (Lyophilisat)

¹H RMN (DMSO), δ(ppm) : 4,9 (1H, d) ; 4,8 (1H, d) ; 4,5 (1H, d) ; 4,05 (1H, d) ; 3,8 (1H, d)

3,42 (1H, m) ; 3,35 (2H, m) ;3,15 (1H, m) ; 3,08 (1H, m); 1,95 (1H, m); 1,55 (2H, m); 1,0 (2H, m) ; 0,95 à 0,85 (9H, m).

### ETUDE PHARMACOLOGIQUE

### EXEMPLE A : Mesure de la sécrétion d'insuline in vitro

Ce test est réalisé par incubation statique d'ilôts de Langerhans isolés. De manière pratique, les ilôts de Langerhans sont isolés du tissu exocrine pancréatique par digestion à la collagénase. Après sélection, les ilôts sont incubés dans un milieu glucosé à 37°C. La quantité d'insuline libérée est mesurée par un dosage radio-immunologique.

### 1 - Isolation des ilôts

Les animaux sont des rats Sprague Dawley (Ch. River) de 250-350 g, non à jeun, anesthésiés au pentobarbital sodique (Nembutal, 40 mg/kg, I.P.).

Une laparotomie médiane est pratiquée afin d'obturer par ligature le canal cholédoque au niveau du duodénum. Un cathéter est introduit dans le canal au niveau hépatique.

Le pancréas est distendu par 20 ml de milieu de Hank's à 4°C à l'aide d'une seringue reliée au cathéter. Le tissu pancréatique est rapidemment prélevé, rincé et débarrassé de la graisse adhérente. Il est ensuite transféré dans un bécher contenant du milieu de Hank's à 4°C et finement découpé pendant 5 à 6 minutes aux ciseaux. Après sédimentation, les fragments tissulaires sont déposés dans un tube stérile de 10 ml en verre siliconé contenant 2 ml de milieu de Hank's dans lequel a été dissoute la collagénase. L'ensemble est agité au vortex pendant 3 minutes sous une lampe équipée d'une ampoule de 100 W. Lorsque la température de 37°C est atteinte, une agitation plus violente (mouvements rapides de va et vient) est maintenue pendant encore 2 ou 3 minutes. La digestion est stoppée par remplissage du tube avec du milieu de Hank's à 4°C et par centrifugation 1 minute à 2500 g. Le surnageant est éliminé. Cette opération est répétée 2 fois. Le dernier culot est repris par 10 ml de milieu de Hank's et observé à la loupe binoculaire.
Les ilôts de Langerhans (100-150 par digestion) les plus denses, aux contours nets et de forme ovoïde, sont prélevés à la pipette Pasteur. Les ilôts encore entourés de tissu pancréatique exocrine témoignent d'une digestion trop douce. Les ilôts ayant un aspect translucide, avec des contours déchiquetés, sont le signe d'une digestion trop agressive.

### 2 - Incubation statique des ilôts

Après sélection, les ilôts sont transférés dans une boite de Pétri contenant du Tampon Krebs Bicarbonate (KRB) pH 7,4, glucosé à 2,8 mM. Les ilôts sont alors répartis par groupe de 10 dans des tubes à hémolyse (13 x 75 mm) préalablement remplis de 2 ml du même tampon. Cette période de préincubation est effectuée dans un bain marie à 37°C, sous flux continu de carbogène pendant 1 heure. L'insuline libérée au cours de cette période est éliminée par lavage dans le tampon KRB glucosé à 2,8 mM.
Les ilôts sont incubés pendant 60 minutes dans 2,5 ml de tampon KRB, la concentration en glucose pouvant être selon le protocole retenu, de 2,8 mM (sécrétion basale), 7 ou 11 mM (sécrétion submaximale) ou 16,7 mM (sécrétion maximale).
Après agitation douce des tubes au vortex, et centrifugation 1 minute à 2500 g, 2 fractions aliquotes de 50 µl sont prélevées pour chaque tube et stockées au congélateur à -20°C.

### 3 - Détermination de la quantité d'insuline sécrétée

La quantité d'insuline lors de l'incubation des ilôts de Langerhans est estimée à l'aide d'un dosage radio-immunologique réalisé avec le Kit Phadeseph Insulin RIA (PHARMACIA). Les ilôts sont incubés à deux concentrations de glucose, l'une basale (2,8 mM) et l'autre simulatrice de la sécrétion d'insuline (16,7 mM). Les résultats sont exprimés en pourcentage de l'effet du glucose. Tous les produits ont été testés à une concentration de 0,1 mM.

**TABLEAU I**

| **Effets des composés sur la sécrétion d'insuline des ilôts de Langerhans** **isolés des rats normaux Sprague Dawley** | | |
|---|---|---|
| **COMPOSES** | **GLUCOSE** | |
| | **2,8 mM** | **16,7 mM** |
| Ex. 2 | -60,5 | -22,6 |
| Ex. 3 | -18,3 | 43,9 |
| Ex. 6 | -5,7 | -70,4 |
| Ex. 13 | -33,3 | -24,8 |
| Ex. 19 | -16,1 | 0,8 |
| Ex. 20 | -59,7 | 1,5 |
| Ex. 22 | -30,8 | -29,0 |
| Ex. 26 | -15,7 | 18,3 |
| Ex. 30 | -44,6 | -0,9 |
| Ex. 31 | -33 | -14,5 |
| Ex. 33 | 19,4 | 17,3 |
| Ex. 34 | -4,1 | 14,4 |

## Revendications

1. Composés de formule (I) : 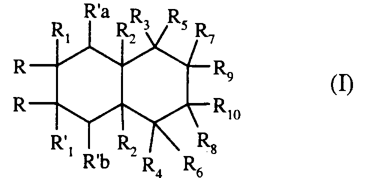 dans laquelle :
- R représente un radical choisi parmi hydroxy, alkoxy et acyloxy,
- R₁, R'₁ et R₂ sont choisis indépendamment l'un de l'autre parmi l'hydrogène et un radical alkyle, les deux groupements R₂ étant en position "cis" par rapport aux cycles,
- R₃ et R₄ représentent l'hydrogène ou forment ensemble un pont méthylène ou éthylène,
- R₅ et R₆, indépendamment l'un de l'autre sont choisis parmi l'hydrogène, le radical hydroxy, un radical alkoxy, un radical acyloxy et un radical alkyle,
- R₇ et R₈, indépendamment l'un de l'autre, sont choisis parmi l'hydrogène et un radical alkyle,
- R₉, R₁₀, R'ₐ et R'_{b}
- sont choisis, indépendamment les uns des autres, parmi l'hydrogène, le radical hydroxy, un radical alkoxy et un radical acyloxy,
- ou bien R₉ et R₁₀ forment ensemble une double liaison ou un cycle avec un atome d'oxygène et R'ₐ et R'_{b} sont tels que définis précédemment,
- ou bien R₉ et R'ₐ forment ensemble un cycle avec un atome d'oxygène et R₁₀ et R'_{b} sont tels que définis précédemment,
- ou bien R₁₀ et R'_{b} forment ensemble un cycle avec un atome d'oxygène et R₉ et R'ₐ sont tels que définis précédemment,
étant entendu que les termes "alkyle", "alkoxy" et "acyloxy" désignent des groupements linéaires ou ramifiés, comportant de 1 à 6 atomes de carbone,
leurs stéréoisomères, ainsi que leurs éventuels sels d'addition à une base, pharmaceutiquement acceptables.

2. Composés selon la revendication 1 pour lesquels R₂ représente l'hydrogène,
leurs stéréoisomères, ainsi que leurs éventuels sels d'addition à une base, pharmaceutiquement acceptables.

3. Composés selon la revendication 1 pour lesquels R₁ représente l'hydrogène,
leurs stéréoisomères, ainsi que leurs éventuels sels d'addition à une base, pharmaceutiquement acceptables.

4. Composé selon l'une quelconque des revendications 1 à 3 qui est le 1,2,3,4,4aβ,5,6,7,8,8aβ-décahydro-2,3,5,6,7,8-hexahydroxy- 1 ,4-éthano-naphtalène,
ses stéréoisomères, ainsi que ses éventuels sels d'addition à une base, pharmaceutiquement acceptables.

5. Composé selon l'une quelconque des revendications 1 à 3 qui est le 1,4,4aβ,5,6,7,8,8aβ-octahydro-5,6,7,8-tétrahydroxy- 1 ,4-éthanonaphtalène,
ses stéréoisomères, ainsi que ses éventuels sels d'addition à une base, pharmaceutiquement acceptables.

6. Composé selon l'une quelconque des revendications 1 à 3 qui est le 1,4,4aβ,5,6,7,8,8aβ-octahydro-1,6,7,8-hexahydroxynaphtalène,
ses stéréoisomères, ainsi que ses éventuels sels d'addition à une base, pharmaceutiquement acceptables.

7. Composé selon l'une quelconque des revendications 1 à 3 qui est le 1,2,3,4,4aβ,5,6,7,8,8aβ-décahydro-2,3-époxy-5,6,7,8-tétra-acétoxy- 1 ,4-éthano-naphtalène,
ses stéréoisomères, ainsi que ses éventuels sels d'addition à une base, pharmaceutiquement acceptables.

8. Composé selon l'une quelconque des revendications 1 à 3 qui est le 1,2,3,4,4aβ,5,6,7,8,8aβ-décahydro-2,6,7,8-tétrahydroxy-3,5-époxyméthano-1-méthyl-4-isopropyl-1,4-éthanonaphtalène,
ses stéréoisomères, ainsi que ses éventuels sels d'addition à une base, pharmaceutiquement acceptables.

9. Composé selon l'une quelconque des revendications 1 à 3 qui est le 1,2,3,4,4aβ,5,6,7,8,8aβ-décahydro-2β,3β,5α,6β,7β,8α-hexahydroxy-1β,4β-éthano-naphtalène,
ainsi que ses éventuels sels d'addition à une base, pharmaceutiquement acceptables.

10. Procédé de préparation des composés de formule (I), caractérisé en ce que l'on engage dans une réaction de Diels-Alder, dans un solvant apolaire, sous atmosphère inerte, à chaud, une quinone de formule (II) et un diène de formule (III) : 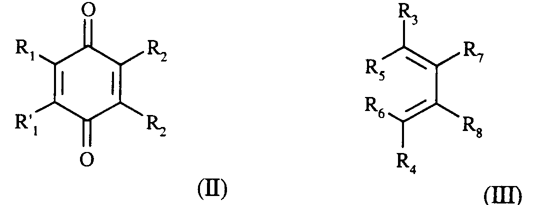 dans lesquelles R₁, R'₁, R₂, R₃, R₄, R₅, R₆, R₇ et R₈ sont tels que définis précédemment,
de manière à obtenir l'intermédiaire de formule (IV) : 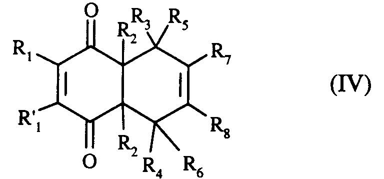 dans laquelle R₁, R'₁, R₂, R₃, R₄, R₅, R₆, R₇ et R₈ sont tels que définis précédemment,
qui, après séparation des éventuels stéréoisomères selon une technique classique de séparation, est ensuite soumis à un agent de réduction, en solvant anhydre, à température appropriée, afin d'obtenir le diol de formule (Va): 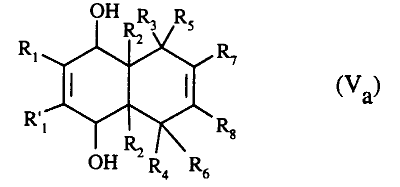 dans laquelle R₁, R'₁, R₂, R₃, R₄, R₅, R₆, R₇ et R₈ sont tels que définis précédemment,
qui, après séparation des éventuels stéréoisomères selon une technique classique de séparation, peut être soumis à l'action d'un agent d'oxydation, pour conduire aux composés de formule (Vₐ₁) et (Vₐ₂), séparés selon une technique classique de séparation: 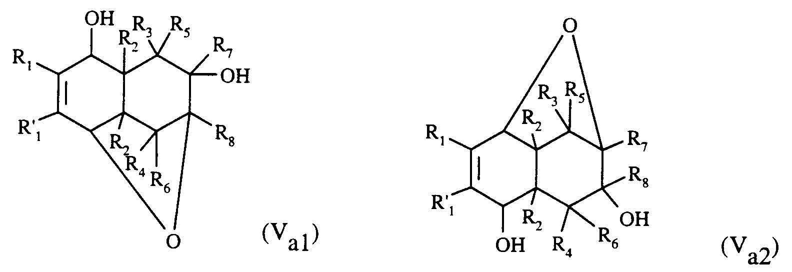 dans lesquelles R₁, R'₁, R₂, R₃, R₄, R₅, R₆, R₇ et R₈ sont tels que définis précédemment,
composés de formules (Vₐ), (Vₐ₁) et (Vₐ₂) dont les fonctions hydroxy peuvent être alkylées ou acylées selon des techniques classiques d'alkylation ou d'acylation respectivement,
les composés de formules (Vₐ), (Vₐ₁) et (Vₐ₂) ainsi que leurs éventuels produits d'alkylation et d'acylation étant ensuite hydroxylés,
- dans le cas d'une cis-hydroxylation par osmylation ou oxydation par le permanganate de potassium, dans les solvants polaires,
- dans le cas d'une trans-hydroxylation par une réaction d'époxydation, suivie d'un traitement en milieu acide,
pour conduire respectivement, après séparation des éventuels stéréoisomères selon des techniques classiques de séparation, aux composés de formules (VIₐ), (VIₐ₁) et (VIₐ₂) : 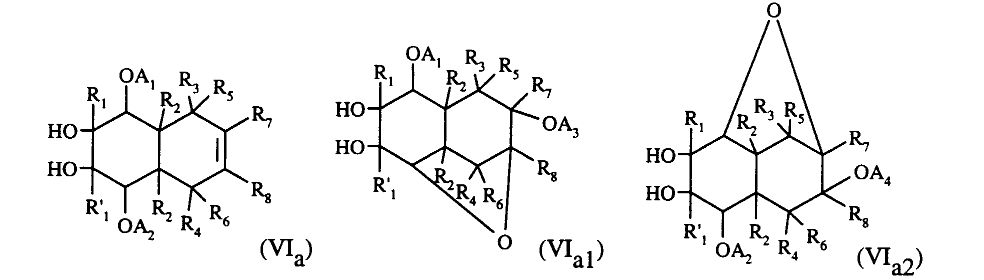 dans laquelle R₁, R'₁, R₂, R₃, R₄, R₅, R₆, R₇ et R₈ sont tels que définis précédemment, et A₁, A₂, A₃ et A₄ représentent indépendamment les uns des autres l'hydrogène ou un groupement alkyle ou acyle tels que définis précédemment,
dont les fonctions hydroxy peuvent être alkylées ou acylées selon des méthodes classiques, l'ensemble des composés de formules (VIₐ), (VIₐ₁) et (VIₐ₂) ainsi que leurs produits d'alkylation et d'acylation formant respectivement l'ensemble des composés de formules (VI) (VI₁) et (VI₂) : 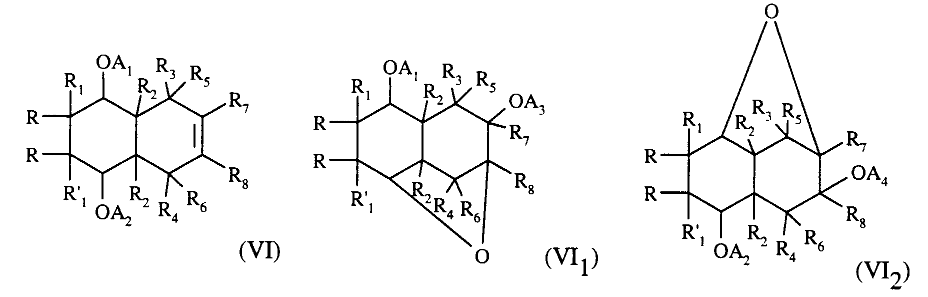 dans lesquelles R, R₁, R'₁, R₂, R₃, R₄, R₅, R₆, R₇, R₈, A₁, A₂, A₃ et A₄ sont tels que définis précédemment,
les composés de formule (VI) pouvant être:
- soit hydrogénés, en présence de catalyseur, en composés de formule (VI_{b}) : 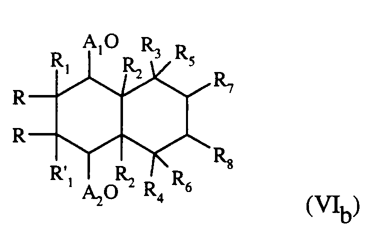 dans laquelle R, R₁, R'₁, R₂, R₃, R₄, R₅, R₆, R₇, R₈, A₁ et A₂ sont tels que définis précédemment,
- soit hydroxylés, en composés de formule (VI_{C1}) : 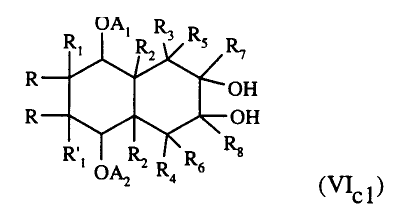 dans laquelle R, R₁, R'₁, R₂, R₃, R₄, R₅, R₆, R₇, R₈, A₁ et A₂ sont tels que définis précédemment,
dont les fonctions hydroxy peuvent être alkylées ou acylées selon des technique classiques, les composés de formule (VI_{C1}) ainsi que leurs produits d'acylation e d'alkylation formant l'ensemble des composés de formule (VI_{C}) : 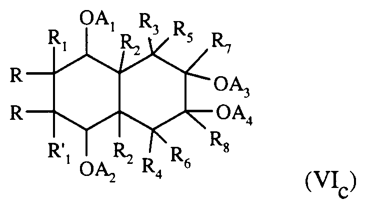 dans laquelle R, R₁, R'₁, R₂, R₃, R₄, R₅, R₆, R₇, R₈, A₁, A₂, A₃ et A₄ sont tels que définis précédemment,
- soit mis en présence d'un agent oxydant, afin d'obtenir le composé de formule (VI_{d}): 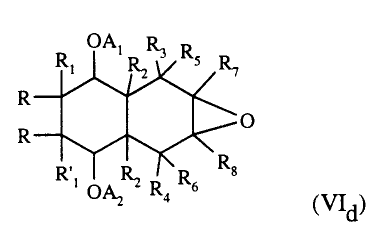 dans laquelle R, R₁, R'₁, R₂, R₃, R₄, R₅, R₆, R₇, R₈, A₁ et A₂ sont tels que définis précédemment,
l'ensemble des composés de formules (VI), (VI₁), (VI₂), (VI_{b}), (VI_{c}) et (VI_{d}) formant l'ensemble des composés de formule (I) qui sont si on le souhaite, purifiés selon une technique classique de purification, et séparés en leurs stéréoisomères éventuels selon des techniques classiques de séparation et transformés en leurs éventuels sels d'addition à une base, pharmaceutiquement acceptables.

11. Compositions pharmaceutiques contenant comme principe actif au moins un composé selon l'une quelconque des revendications 1 à 9, seul ou en combinaison avec un ou plusieurs excipients inertes, non toxiques et pharmaceutiquement acceptables.

12. Compositions pharmaceutiques selon la revendication 11 régulant la sécrétion d'insuline et utiles dans le traitement du diabète, de l'insulinorésistance, des maladies cardiovasculaires, et des insulinomes.
